(19) Europäisches Patentamt
European Patent Office
Office européen des brevets

(11) **EP 0 680 605 B1**

(12) **EUROPEAN PATENT SPECIFICATION**

(45) Date of publication and mention
of the grant of the patent:
**30.01.2002 Bulletin 2002/05**

(21) Application number: **95904142.7**

(22) Date of filing: **22.11.1994**

(51) Int Cl.[7]: **G01N 27/447**, C12Q 1/68

(86) International application number:
**PCT/US94/13605**

(87) International publication number:
**WO 95/14922 (01.06.1995 Gazette 1995/23)**

(54) **ENTRAPMENT OF NUCLEIC ACID SEQUENCING TEMPLATE IN SAMPLE MIXTURES BY ENTANGLED POLYMER NETWORKS**

EINSCHLIESSEN VON MATRIZEN ZUR SEQUENZIERUNG VON NUKLEINSAEUREN IN PROBENMISCHUNGEN BEI VERFLOCHTENEN POLYMERNETZWERKEN

CAPTURE D'UNE MATRICE DE SEQUEN AGE D'ACIDES NUCLEIQUES DANS DES MELANGES D'ECHANTILLONS PAR DES RESEAUX DE POLYMERE ENCHEVETRE

(84) Designated Contracting States:
**AT BE CH DE DK FR GB IT LI LU NL SE**

(30) Priority: **23.11.1993 US 156218**

(43) Date of publication of application:
**08.11.1995 Bulletin 1995/45**

(73) Proprietor: **PE Corporation (NY)**
**Foster City California 94404 (US)**

(72) Inventor: **JOHNSON, Ben, F.**
**Palo Alto, CA 94303 (US)**

(74) Representative: **West, Alan Harry et al**
**R.G.C. Jenkins & Co. 26 Caxton Street**
**London SW1H 0RJ (GB)**

(56) References cited:
EP-A- 0 162 657      EP-A- 0 459 241
EP-A- 0 471 949      EP-A- 0 497 448
EP-A- 0 500 211      US-A- 4 911 807

## Description

This invention generally relates to capillary electrophoresis of nucleic acids such as DNA, and more particularly to a sample preparation technique to restrict the mobility of nucleic acid templates in a sample solution.

Gel electrophoresis is a powerful method of separating large biomolecules, such as proteins, deoxyribonucleic acids (DNA), and ribonucleic acids (RNA). In gel electrophoresis, a mixture of biomolecules is placed on a selected gel medium and the gel is subjected to an external electric field. The velocity (v) of migration of a biomolecule through the gel depends on the strength of the electric field (E), the net charge (z) on the molecule, and the frictional coefficient (f) of the medium:

$$V = Ez/f$$

The frictional coefficient depends on the mass and shape of the molectule, the viscosity, and the porosity of the medium.

Gels have become the preferred medium for conducting electrophoretic separations because they suppress the convective currents produced by small temperature gradients in less viscous media, and they act as molecular sieves which inhibit movement of large molecules, but permit smaller molecules to move readily through the pores of the gel. Polyacrylamide gels have generally been the medium of choice for performing separations because they are chemically inert and their pore sizes can be controlled by selection of a desired ratio of acrylamide and methylenebisacrylamide (cross-linking agent), and total monomer concentrations used in polymerization. The polyacrylamide gel is typically generated by free radical polymerization of the component monomers, using a free radical initiator, in the presence of the electrophoresis medium.

Electrophoretic separations of proteins are often performed in a cross-linked polyacrylamide gel under protein denaturing conditions. For example, proteins can be dissolved in a detergent solution, e.g., sodium dodecyl sulfate (SDS), and subjected to mercaptoethanol or dithiothreitol treatment to reduce any disulfide bonds. The SDS anions bind to the protein at a ratio of about one SDS molecule to two amino acid residues, thereby imparting a large net negative charge and bulk to the denatured protein. The charge and bulk of the protein-SDS complex are roughly proportional to the mass of the native protein. Displacements of a protein or peptide within a gel matrix can thereby be related to molecular size on a basis of the size and charge on the molecule. In the case of nucleic acids, which have roughly the same charge density, displacement in the gel matrix is more directly related to molecular size.

Electrophoresed complexes are usually visualized by staining with a dye, such as Coomassie blue, or by autoradiography when the molecules are radioactively labeled. The displacement of a biomolecule in the gel is nearly linearly proportional to the logarithm of the mass of the molecule, with exceptions found for such species as glycosylated and membrane proteins. Proteins differing by as little as 2% in mass can often be distinguished by electrophoresis.

One electrophoretic technique that permits rapid, high-resolution separation is capillary electrophoresis (CE). In one CE procedure, a capillary tube is filled with a fluid electrophoresis medium and the fluid medium is crosslinked or temperature-solidified within the tube to form a non-flowable, stabilized separation medium. A sample volume is drawn into one end of the tube by electrokinetic injection, and an electric field is applied across the tube to draw the sample through the medium. Typically, a bioseparation conducted by CE employs fused silica capillary tubes having inner diameters between about 50-200 microns, and ranging in length between about 10-100 cm or more.

The polymer concentration and/or degree of cross-linking of the separation medium may be varied to provide separation of species over a wide range of molecular weights and charges. For example, in separating nucleic acid fragments greater than about 1,000 bases, one preferred temperature-solidified material is agarose, where the concentration of the agarose may vary from about 0.3%, for separating fragments in the 5-60 kilobase size range, up to about 2%, for separating fragments in the 100-3,000 basepair range. Smaller size fragments, typically less than about 1,000 basepairs, are usually separated in cross-linked polyacrylamide. The concentration of acrylamide polymer can range from about 3.5%, for separating fragments in the 100-1,000 basepair range, up to about 20%, for achieving separation in the 10-100 basepair range. For separating proteins, cross-linked polyacrylamide at concentrations between about 3-20% are generally suitable. In general, the smaller the molecular species to be fractionated, the higher is the concentration of cross-linked polymer required.

The resolution obtainable in solidified electrophoresis media of the type described above has been limited, in the case of small molecular weight species, by difficulties in forming a homogeneous, uniform polymer matrix at high polymer concentration within an electrophoresis tube, and especially within a capillary tube. In one general method for forming a high-concentration solidified matrix in a tube, a high-concentration polymer solution, in a non-crosslinked, low-viscosity form, is introduced in fluid form into the tube. The fluid material is then cross-linked, for example, by exposure to light in the presence of persulfate and a cross-linking agent.

At high polymer concentrations, polymerization reaction heat gradients formed within the tube tend to produce uneven rates of reaction and heat turbulence which can lead to matrix inhomogeneities. Also, entrapped gas bubbles generated during the crosslinking reaction produce voids throughout the matrix. The non-uniformities in the matrix limit the degree of resolution that can be achieved, particularly among closely related, small molecular weight species. These problems may be overcome by polymerizing the gel material at elevated pressure; however, producing a controlled pressure within a capillary gel introduces difficult technical problems.

In the case of temperature-solidified gels, a polymer is introduced into an electrophoresis tube in a fluid form, then allowed to gel to a solid form by cooling within the tube. This approach, however, is generally unsuitable for fractionating low molecular weight species, such as small peptides and oligonucleotides, since the polymers, such as agar and agarose, that are known to have the necessary temperature-solidifying setting properties are not effective for fractionating low molecular weight species, even at high polymer concentrations.

A second limitation associated with crosslinked or temperature-solidified matrices is the difficulty in removing crosslinked gel matrix from the gel support. In the case of a capillary-tube support, this may prevent recovery of separated material within the gel, and also may prevent reuse of the capillary tube.

The gel matrix employed in capillary electrophoretic systems has historically generally been a solid gel such as an agarose gel or cross-link polymer matrix, such as a cross-link polyacrylamide matrix. Such gels nay be difficult to introduce into the capillary tube without bubbles or voids, and generally preclude reusing the tube. More recently, capillary electrophoresis systems employing a polymer solution as separation medium have been disclosed. U.S. Patent No. 5,096,554, entitled "Nucleic Acid Fractionation by Counter Migration Capillary Electrophoresis", describes an electrophoresis system in which DNA fractionation occurs in a polymer solution which itself is migrating through the tube by electroosmotic flow in a direction opposite to that of DNA movement in the gel. Another co-owned U.S. Patent, No. 5,164,055, for "High Viscosity Polymer Matrix and Methods", discloses the use of a viscoelastic polymer solution as a substitute matrix for a cross-link gel matrix in capillary electrophoresis. U.S. Patent, No. 5,126,021, entitled "Low viscosity Polymer Solution for Capillary Electrophoresis", discloses a capillary electrophoresis tube containing a low viscosity polymer solution having a selected mesh size and low solution viscosity. Mesh size may range from 50-100 angstrom, for separating single-stranded oligonucleidas; to up to 300 angstrom or greater for separating relatively *large* duplex DNA fragments or proteins.

More recently, a viscous electrophoresis polymer medium has been developed which is a stabilized gel, easily removed from the capillary tubes, which comprises a matrix of aggregated regular, alternating copolymers in an aqueous medium. The copolymers are composed of hydrophilic polymer segments having selected substantially uniform sagment links in a plurality of hydrophobic polymer segments carried on in space from one another at regular repeating intervals by the hydrophilic polymer segments. This medium is characterized by 1) the ability of the medium to effect a high resolution electrophoretic separation of biopolymer molecules in a defined molecular size range; and 2) a concentration of the copolymer which is above the interpolymeric aggregation transition concentration defined by the concentration of copolymer at which a marked rise in viscosity of an aqueous dispersion of the copolymer is observed. The copolymers may have a comb or tuft structure, a block structure, or a star structure, depending on how the hydrophobic polymer chains are arranged.

Electrokinetic loading of a liquid nucleic acid sequencing sample mixture containing nucleic acid target, template and partial-sequence nucleic acid fragment analytes such as DNA extension products into a capillary electrophoresis tube filled with a gel medium such as an agarose gel or polymer gel as described above is the preferred method of introducing a sample of analytes into the capillary electrophoresis tube. Electrokinetic loading preferentially introduces the analytes and thus, in effect, concentrates the sample. However, the amount of analytes introduced into the capillary electrophoresis medium is limited by nucleic acid template buildup on the injection end surface of the CE medium in the capillary tube. This template buildup clogs the end of the capillary tube with these large biomolecules and prevents passage of additional analytes into the medium. This phenomena effectively limits the maximum amount of partial-sequence fragments that can be injected and electrophoresed.

This clogging problem is especially severe in capillary electrophoresis, since clogging of the end of the capillary not only blocks entry of sample components, but also causes a series cf events that result in extensive bubble formation in the capillary tube which interferes with both the resolution of extension products and the electrical conductivity of the capillary.

For example, the maximum injection time before clogging of a conventional CE capillary tube filled with a comb polymer gel medium, is about 60 seconds at 0.7 kV (0.4 IAA) which is equivalent to a seconds at 4.5 kV. This clogging of the capillary electrophoresis tube in turn severely limits the amount of extension product (partial-sequence fragments) that can be resolved during capillary electrophoresis.

One solution to clogging of the end of the capillary tube is to effectively eliminate the template DNA from the sample by dipyramidination with UDG enzyme. This method is described in Swerdlow et al, "Stability of Capillary Gels for Automated Sequencing of DNA", *Electrophoresis* 1992, 13, 475-483. UDG is an enzyme which edits DNA to eliminate occasional uracil residues which may be inadvertently incorporated by DNA polymerase, or produced by diamination of cytosine.

Another solution to clogging of the capillary electrophoresis tube is to cut off the template clogged end of the capillary tube shortly after introduction of the sample. The cut end thus presents a new end surface for introducing the buffer and\or analytes from the run underway as well as for the next sample to be introduced into the tube. This step is unsatisfactory in that only a few samples can be run sequentially through the same capillary tube before the shortening of the capillary length adversely affects resolution and reproducibility of DNA fragment separation. Alternatively, a new tube may be utilized for each sample.

The present invention seeks to provide a method of increasing the amount of analyte or analytes in the liquid sample that can be introduced into the entrance end of the capillary electrophoresis tube.

The present invention seeks also to provide a method of retarding the migration of large macromolecules in the sample solution, thereby lengthening the time during which smaller biomolecules may be introduced into the electrophoretic medium, and to provide capillary electrophoresis apparatus which enhances the resolution of partial-sequence DNA fragments by having the sample mixture embedded in an entangled polymer matrix effective to retard movement of the template DNA during electrokinetic introduction of the partial-sequence DNA fragments into the capillary tube.

According to a first aspect of the invention, there is now provided a method for electrophoretically separating biomolecules comprising:

a) providing a mixture of biomolecule analytes and macrobiomolecules having a greater molecular weight than said biomolecular analytes in a solvent;
b) embedding the mixture in a polymer matrix effective to preferentially retard the movement of the macrobiomolecules through the matrix, when an electric field is placed across the matrix;
c) placing the matrix having the embedded mixture therein in communication with one end region of an elongate electrophoretic medium effective to resolve such biomolecular analytes, when an electric field is placed across the medium;
d) applying an electric field across the matrix and the medium, in a direction which draws the biomolecule analytes through the matrix and into and through the medium, whereby an increase in the proportion of biomolecule analytes relative to macrobiomolecules entering the electrophoresis medium is achieved.

In accordance with a further aspect of the invention there is provided also a method of entrapping DNA sequencing template macromolecules in a sample mixture comprising the steps of:

a) providing a sequencing sample mixture containing at least a DNA template macromolecule having a molecular weight greater than about $6 \times 10^5$ Daltons, a DNA extension product, and a solvent, and
b) introducing a linear polymer into the mixture formulating an open entangled polymer network in the mixture embedding the DNA template and DNA extension products therein wherein the network has sieving properties effective to retard movement of the DNA template.

Additionally, there is provided a method of loading a sample into a capillary tube for capillary electrophoresis comprising the steps of:

a) providing a sample mixture containing macromolecules and analytes of interest in a solvent;
b) introducing into the mixture a linear polymer having a molecular weight effective to form an entangled polymer matrix embedding said sample mixture therein, said matrix having a mesh size effective to retard movement of the macromolecules through the matrix when an electric field is applied across the matrix;
c) inserting one end of a separation medium filled capillary electrophoresis tube into the sample mixture and matrix;
d) applying an electrical potential across the sample mixture and the medium effective to increase the proportion of analytes of interest relative to macrobiomolecules entering the electrophoresis medium.

Still further, there is provided a capillary electrophoresis sample for electrophoretic injection into a capillary electrophoresis separation medium, said sample comprising macromolecules and analytes of interest in solution embedded in an entangled polymer matrix having a mesh size effective to retard movement of said macromolecules within said sample through the matrix when an electric field is applied across the matrix to draw said analytes through said matrix.

Also, there is provided a method for sequencing a nucleic acid fragment comprising:

a) providing a mixture of primer extension fragments in a fragment mixture also containing template nucleic acid molecules;
b) embedding the fragment mixture in a polymer matrix effective to preferentially retard the movement of the template nucleic acid molecules through the matrix, when an electric field is placed across the matrix;
c) placing the matrix and embedded mixture in communication with one end region of an elongate electrophoretic medium effective to resolve such primer extension fragments, when an electric field is placed across the medium;
d) applying an electric field across the matrix and the medium, in a direction which draws primer extension fragments through the matrix and into and through the medium, whereby an increase in the proportion of primer extension fragments relative to template entering the electrophoresis medium is realized.

Still further, there is provided apparatus for use in sequencing a nucleic acid fragment by electrophoretic separation of a mixture of primer extension fragments in a fragment mixture also containing template nucleic acids, said apparatus comprising:

a polymer matrix effective to preferentially retard the movement of the template nucleic acids through the matrix, when such a mixture is embedded in the matrix and an electric field is placed across the matrix;

an elongate electrophoretic medium effective to resolve such primer extension fragments, when an electric field is placed across the medium said medium having one end in communication with said matrix; and

means for applying an electric field across the matrix and the medium, in a direction which draws the primer extension fragments through the matrix and into and through the medium.

This means may be a constant voltage or a pulsed voltage sowel, as is generally used in capillary electrophoresis.

In general terms, the methods of the invention involve introducing a small concentration of a long linear polymer solution into the DNA sequencing sample mixture before electrokinetic loading or injection of the analytes into the capillary tube. This long linear polymer solution creates an open entangled polymer network or matrix as described in U.S. Patent No. 5,126,021 into which the sample mixture containing the DNA template macrobiomolecule and bionolecules such as DNA extension products integrates or becomes embedded. This open entangled polymer network retards the mobility of the DNA template macrobiomolecules while effectively allowing free passage of the smaller biomolecules such as the partial-sequence fragments e.g. DNA extension products. In effect, the open entangled polymer network or matrix has sieving properties that preferentially restricts movement of large biomolecules having a size greater than about 2000 bases or base-pairs (bp). The long linear polymer selected is preferably hydroxyethylcellulose (HEC) with a molecular weight of between $2 \times 10^5$ and $5 \times 10^6$ Daltons. Chemically similar polymers may also be utilized.

Preferably the DNA sequencing sample mixture includes a denaturant sufficient to denature double-stranded DNA at room temperature, i.e., to render the double-stranded DNA single stranded. Preferred denaturants include urea, dimethylformamide, n-methyl-2-pyrrolidinone, and 2-pyrrolidinone. More preferably, the denaturant is 2-pyrrolidinone. The denaturant concentration is preferably between 10 and 70% by weight.

The apparatus and method in accordance with the invention basically entraps macrobiomolecules in a sample mixture containing macrobiomolecules and biomolecule analytes of interest in a solvent by introducing into the mixture a linear polymer having a molecular weight effective to form an entangled polymer network into which the sample mixture is embedded. The network or matrix has a mesh size effective to retard movement of the macrobiomolecules through the matrix when an electric field is applied in a direction to draw the biomolecules in one direction through the matrix.

The macrobiomolecules include proteins and nucleic acid sequencing templates and generally have a molecular weight of at least 5000 Daltons. For DNA sequencing templates of at least about 2000 bases or base-pairs, the molecular weight is at least about $6 \times 10^5$ Daltons.

The present invention is particularly suited for sequencing of nucleic acid fragments electrophoretically in an elongated separation medium such as a gel in a capillary tube.

The invention is described below in greater detail by way of example only with reference to the accompanying drawings, in which

Figure 1 is an electropherogram of the results of control Example 1;

Figure 2 is a first part of the electropherogram of the results of Example 2;

Figure 3 is the second part of an electropherogram of the results of Example 2;

Figure 4 is an electropherogram of the results of Example. 3;

Figure 5 is an electropherogram of the results of Example 4; and

Figure 6 is a simplified schematic view of a system in accordance with the invention.

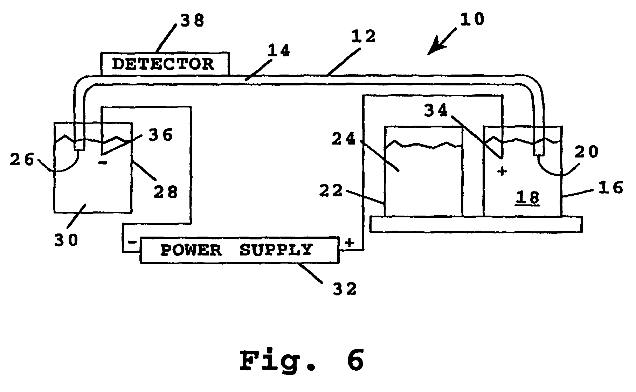

A simplified schematic view of a cappilary electrophoresis apparatus suitable for use in practicing the method of the invention is shown in Figure 6. The system 10 includes a capillary-tube 12 supporting a separation medium 14. This medium may be an entangled polymer, a gel, or any other separation medium such as has been previously described. An anodic container or reservoir 16 in the system contains an electrolytic solution 18. The anodic end of the tube, indicated at 20, is immersed in the sample solution, as shown, during electrophoresis. A reservoir 22 in the system may contain a marker solution, or may contain a sample solution 24 of biomolecules to be separated, during an electrophoretic separation. This sample solution includes the entangled polymer matrix to retard movement of the large macrobiomolecules in the sample. The two anodic reservoirs may be carried on a carousel or the like, for placement at a position in which the lower anodic end 20 of the tube 12 can be immersed in the reservoir fluid (18 or 24). Although not shown here, the carousel may carry additional reservoirs containing solutions for cleaning and flushing the tube between electrophoretic runs or different solutions, where two or more solutions are employed in a single electrophoretic fractionation method.

The opposite, cathodic end 26 of the tube 12, is sealed within a cathodic reservoir 28 and is immersed in an cathodic electrolyte solution 30 contained in the reservoir 28, as shown.

A high voltage supply 32 in the system 10 is connected to the anodic and cathodic reservoirs 18 and 28 as shown, for applying a selected electric potential between the two reservoirs. The power supply leads are connected to platinum electrodes 34, 36 in the anodic and cathodic reservoirs, respectively. The power supply may be designed for applying a constant voltage (DC) across the electrodes, preferably at a voltage setting of between 5-50 KV. Alternatively, or in addition, the power supply may be designed to apply a selectedfrequency, pulsed voltage between the reservoirs. In general, the shorter the capillary tube, the higher the electric field strength that can be applied, and the more rapid the electrophoretic separation.

When operated in a pulsed voltage mode, the power supply preferably outputs a square wave pulse at an adjustable frequency of about 50 Hz up to a KHz range, and an rms voltage output of about 10-30 KV. Higher pulse frequencies, even into the MHz range may be suitable for some applications.

Completing the description of the system shown in Figure 6, a detector 38 in the system is positioned adjacent the cathodic end of the tube, for optically monitoring nucleic acid fragments migrating through an optical detection zone 40 in the tube. The detector may be designed either for UV absorption detection and/ or for fluorescence emission detection. UV absorbance is typically carried out at 205-280 nm, using, for example, a Kratos 783 UV absorbance detector which has been modified by Applied Biosystems (Foster City, CA.), by replacing the flow cell with a capillary holder. Fluorescence emission detection is preferably carried out at a selected excitation wavelength which is adjustable between about 240-500 nm, depending on the fluorescent species associated with the nucleic acid fragments, as discussed below. one exemplary fluorescence detector is an HP1046A detector available from Hewlett-Packard (Palo Alto, CA) , and modified as above for capillary tube detection. The detector is connected to an integrator/ plotter 45 for recording electrophoretic peaks.

One preferred example of a sample solution containing a sample mixture embedded in an entangled polymer matrix in accordance with the invention is a sample mixture containing DNA templates and extension products, a long linear polymer such as hydroxyethyl cellulose (HEC) with a molecular weight of about 4 x $10^6$ Daltons (such as Union Carbide QP10OMH) dissolved in a solvent comprising 2-pyrrolidinone, water, and 2.5 mM EDTA adjusted to a minimal value that restricts electrophoretic mobility of the DNA template. An effective minimal concentration of the linear polymer in the solution mixture is between 0.1 to about 0.2 percent This concentration results in successful injection times of at least 40 seconds at 4. 5 Kv. This is a factor of a increase over conventional electrokinetic injection times. A preferred solvent is between 10%(wt/wt) and 70%(wt) 2-pyrrolidinone in water.

The following examples illustrate the invention.

### Example 1

Atype DB-1 capillary tube, obtained from J and W Scientific, Folsom, CA, Catalog No. 126-1013, was prepared and cut into 50 centimeter lengths. The tubing had an internal diameter of 50 μm. The capillary was then rinsed with methanol and water. The capillary was then hydrodynamically filled with a polyethylene glycol (PEG) / fluorinated copolymer gel consisting of 7% C4F9/Carbowax 4600 in 125 mM borate - tetramethyl ammonium hydroxide (TMA), 1.25 mM of EDTA, 6.6 molar urea and a pH of 9.0 at standard conditions. The 50 cm capillary tube was filled half full in 8 minutes, and fully filled in 32 minutes.

The first sample was a control sample, without the entangled polymer in the sample solution. A single color sequencing ladder of fragments terminating at c was prepared by the dideoxy sequencing method using a sequencing kit and accompanying protocols from Applied Biosystems (part No. 401119). An M13mp18 DNA template (m13mp18(÷)strand, 0.1 pmole) was annealed to a fluorescent dye primer (FAM M13 (-21) primer, and primer extension was carried out using Taq polymerase, with dideoxycytidine provided as the 31-terminating base.

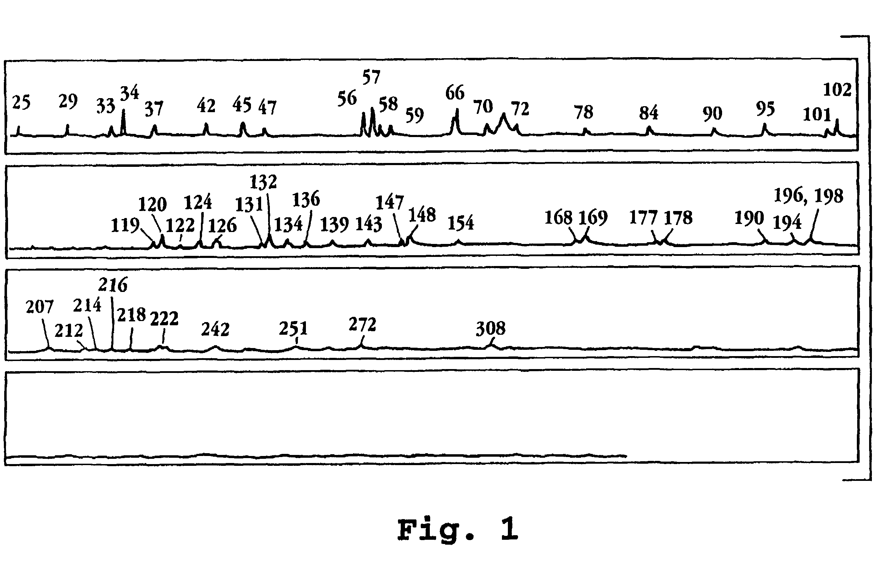

The sample was prepared in a vial containing 5 μl of formamide plus 2.5 mM sodium EDTA, pH of 9. The sample contained the reactants from FAM Taq M13 (-21) primer sequence of 0.5pg M13 template DNA dissolved in the 5 μl of formamide plus 2.5 mM EDTA. The sample was then heated at 90°C for 2 minutes. Prior to the electrokinetic injection, a preconditioning run was done at 9 kV, 5.8 μA on the tube. The electrokinetic sample injection was performed at 0.4 μA, 0.9 kV, for 60 seconds to achieve a charge total of 24 μCoulombs. The resultant electropherogram is shown in Fig. 1.

### Example 2

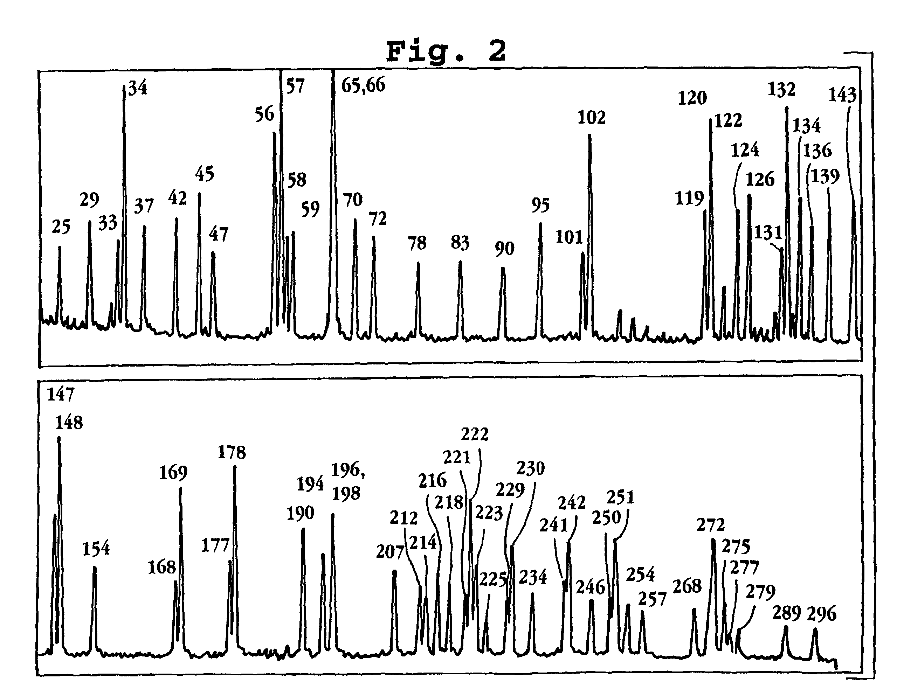

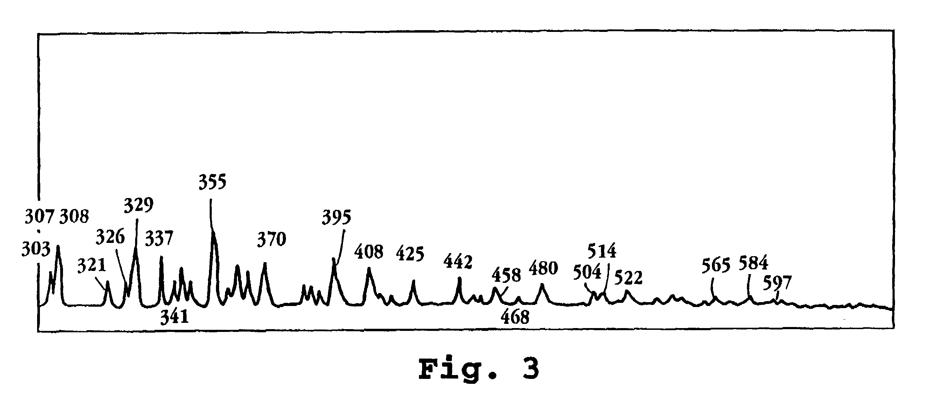

A capillary tube section 50 cm in length and 50 μm in diameter was prepared as above described in Example 1 with a 7% gel made of C4F9/Carbowax 4600 in 125 mM boric acid-TMA, 1.25 mM EDTA, 6.6 molar urea, and a ph of 9.0. The sample in this case was the reactants from FAM Taq M13 (-21) primer sequence of 0.5μg M13 template DNA dissolved in 5 μl of formamide plus 2.5 mM sodium EDTA, plus 0 - 1% QP100MH HEC (hydroxyethyl cellulose) . The sample solution was heated to 90°C for 2 minutes, and then electrokinetically injected into the capillary tube at 4.5 kV, 3 μA, for 20 seconds. The resultant electropherogram is shown in Figures 2 and 3.

Example 3

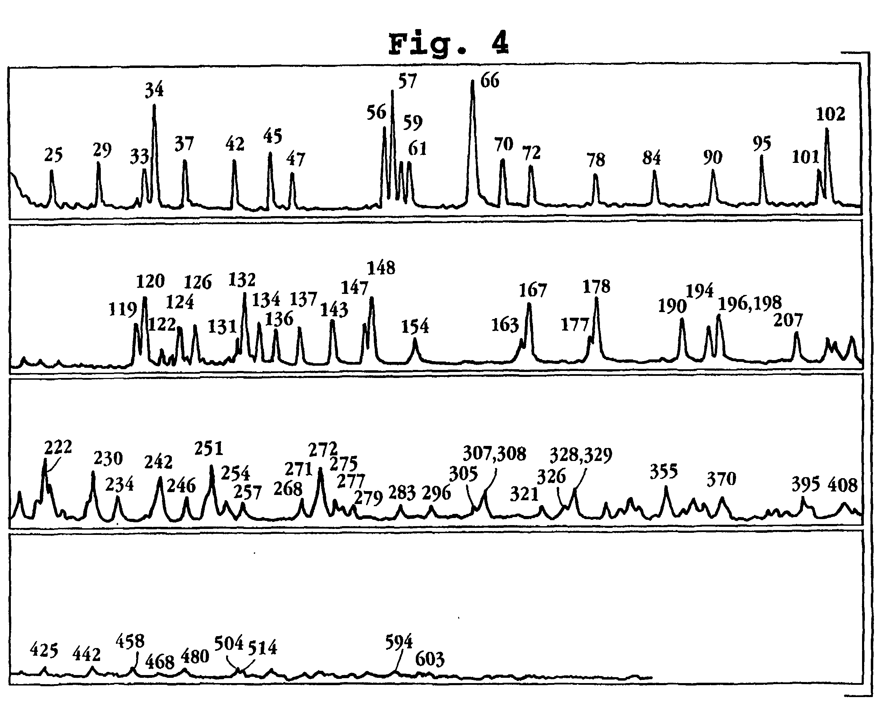

A capillary tube 50 cm in length and 50 μm in diameter was prepared with a 7% gel made of C4F9/Carbowax 4600 in 125 mM borate-TMA, 1.25 mM sodium EDTA, 6.6 molar urea, and a pH of 9.0. The sample in this case was the reactants from FAM Taq M13 (-21) primer sequence of 0.5gg M13 template DNA dissolved in 5 gl of formamide plus 2.5 mM sodium EDTA, plus 0.15% QP100HH HEC. The sample solution was heated to 900C for 2 minutes, and then electrokinetically injected into the capillary tube at 4.5 kV, 3 μA, for 20 seconds. The results of this experiment are shown in Fig. 4.

Example 4

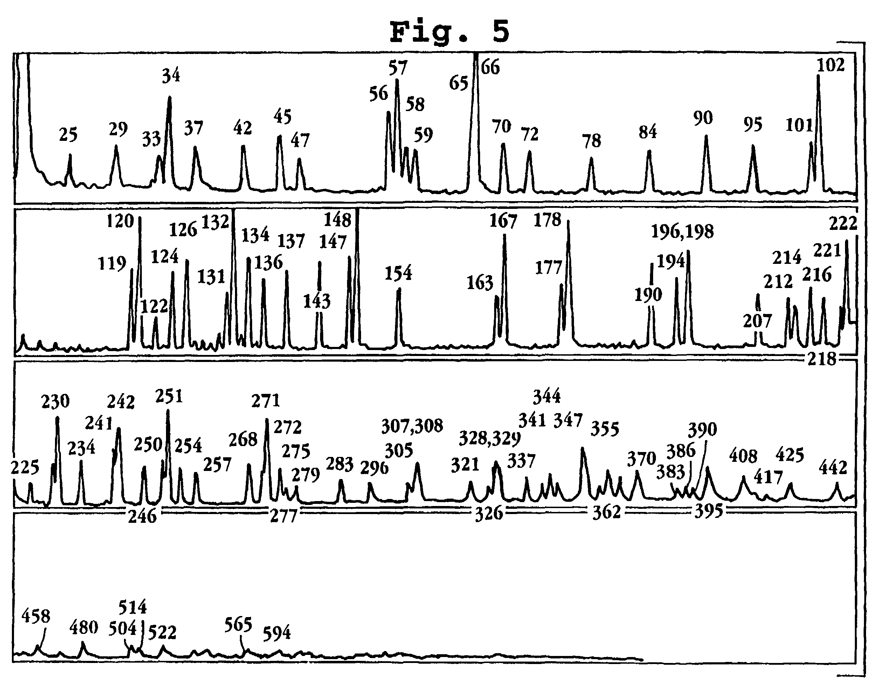

A capillary tube 50 cm in length and 50 μm in diameter was prepared with a 7% gel made of C4F9/Carbowax 4600 in 125 mm borate-TMA, 1.25 mM EDTA, 6.6 molar urea, and a pH of 9.0. The sample in this case were the reactants from FAM Taq M13 (-21) primer sequence of 0.5μg M13 template DNA dissolved in 5 yl of formamide plus 2.5 mM sodium EDTA, plus 0.15% QP100MH HEC- The sample solution was heated to 90°C for 2 minutes, and then electrokinetically injected into the capillary tube at 4.5 kV, 3 μA, for 40 seconds. The results of this experiment are shown in Figure 5.

The electropherograms in Figures 1 through 5 plot signal amplitude versus time. The amplitude of signal is generally proportional to the quantity of analyte injected. The numbers above the peaks indicate the number of basepairs in the segment. The sample mixture in Example 1 was a control which did not contain an entangled polymer as in the other examples. It can be readily seen that the quantity of DNA extension products introduced into the capillary tube is substantially greater in each of Examples 2, 3, and 4, shown in Figures 2 through 5 compared to the control sample injection reflected in Figure 1. The amplitudes in the control electropherogram (Figure 1) are at least about an eighth to a tenth that of the examples containing the QP1000MH HEC entangled polymer.

While the invention has been described with reference to particular embodiments thereof, it should be apparent that the sample composition in the method may be practiced other than as specifically described. Various polymers and copolymers may be utilized to retard or inhibit movement of the DNA sequencing template in the system and method in accordance with the invention provided that the polymers or copolymers form an entangled polymer matrix in which the sample is embedded.

In addition to the polymers above described which may be used in the invention, the concentration of the polymers or copolymers in the sample mixture will affect the mobility of the macromolecules such as the DNA sequencing templates. For example, when a high molecular weight HEC such as QP100MH HEC is utilized, an effective minimum concentration is .1% to .2%. Where a different polymer is used, the concentration must be varied to optimize the mobility restriction without affecting mobility of the analytes of interest.

**Claims**

1. A method for electrophoretically separating biomolecules comprising:

   a) providing a mixture of biomolecule analytes and macrobiomolecules having a greater molecular weight than said biomolecule analytes in a solvent;
   b) embedding the mixture in a polymer matrix effective to preferentially retard the movement of the macrobiomolecules through the matrix, when an 'electric field is placed across the matrix;
   c) placing the matrix having the embedded mixture therein in communication with one end region of an elongate electrophoretic medium effective to resolve such biomolecule analytes, when an electric field is placed across the medium;
   d) applying an electric field across the matrix and the medium, in a direction which draws the biomolecule analytes through the matrix and into and through the medium, whereby an increase in the proportion of biomolecule analytes relative to macrobiomolecules entering the electrophoresis medium is achieved.

2. A method according to claim 1, wherein said macrobiomolecules are DNA sequencing templates.

3. A method according to claim 1 or claim 2 wherein said biomolecule analytes are primer extension fragments.

4. A method according to any one of claims 1 to 3, wherein said macrobiomolecules have a molecular weight of at least 5000 Daltons.

5. A method according to anyone of claims 1 to 4 wherein said polymer is hydroxyethyl cellulose.

6. A method of entrapping DNA sequencing template macromolecules in a sample mixture comprising the steps of:

a) providing a sequencing sample mixture containing at least a DNA template macromolecule having a molecular weight greater than about $6 \times 10^5$ Daltons, a DNA extension product, and a solvent, and

b) introducing a linear polymer into the mixture formulating an open entangled polymer network in the mixture embedding the DNA template and DNA extansion products therein wherein the network has sieving properties effective to retard movement of the DNA template.

7. A method of loading a sample into a capillary tube for capillary electrophoresis comprising the steps of:

a) providing a sample mixture containing macromolecules and analytes of interest in a solvent;

b) introducing into the mixture a linear polymer having a molecular weight effective to form an entangled polymer matrix embedding said sample mixture therein, said matrix having a mesh size effective to retard movement of the macromolecules through the matrix when an electric field is applied across the matrix;

c) inserting one end of a separation medium filled capillary electrophoresis tube into the sample mixture and matrix;

d) applying an electrical potential across the sample mixture and the medium effective to the increase the proportion of the analytes of interest relative to macrobiomolecules entering the electrophoresis medium.

8. A method according to claim 7, wherein said macromolecules are nucleic acid templates.

9. A method according to claim 7 or claim 8, wherein said analytes of interest include DNA extension products.

10. A method according to any one of claims 7 to 9, wherein said matrix is formed of a linear polymer having a molecular weight between $2 \times 10^5$ and $5 \times 10^6$ Daltons.

11. A capillary electrophoresis sample for electrophoretic injection into a capillary electrophoresis separation medium, said sample comprising macromolecules and analytes of interest in solution embedded in an entangled polymer matrix having a mesh size effective to retard movement of said macromolecules within said sample through the matrix when an electric field is applied across the matrix to draw said analytes through said matrix.

12. A sample according to claim 11, wherein said macromolecules are DNA sequencing templates.

13. A sample according to claim 11 or claim 12, wherein said analytes of interest are DNA extension products.

14. A sample according to any one of claims 11 to 13, wherein said entangled polymer matrix is formed of a linear polymer having a molecular weight between $2 \times 10^5$ and $5 \times 10^6$ Daltons.

15. A method for sequencing a nucleic acid fragment comprising:

a) providing a mixture of primer extension fragments in a fragment mixture also containing template nucleic acid molecules;

b) embedding the fragment mixture in a polymer matrix effective to preferentially retard the movement of the template nucleic acid molecules through the matrix, when an electric field is placed across the matrix;

c) placing the matrix and embedded mixture in communication with one end region of an elongate electrophoretic medium effective to resolve such primer extension fragments, when an electric field is placed across the medium;

d) applying an electric field across the matrix and the medium, in a direction which draws primer extension fragments through the matrix and into and through the medium, whereby an increase in the proportion of primer extension fragments relative to template entering the electrophoresis medium is realized.

16. A method according to claim 15 wherein said entangled polymer matrix is formed of a linear polymer having a molecular weight of between about $2 \times 10^5$ and $5 \times 10^6$ Daltons.

17. A method according to any one of claims 3, 6, 9, 15 and 16, wherein the mixture/sample mixture/fragment mixture further includes a denaturant.

18. A method according to claim 17, wherein the denaturant is selected from urea, formamide, 2-pyrrolidinone and n-methyl-2-pyrrolidinone.

19. A method according to claim 17 or claim 18, wherein the denaturant is in aqueous solution having a concentration of 10 to 70% by weight.

20. An electrophoretic apparatus (10) for use in sequencing a nucleic acid fragment, by electrophoretic separation of a mixture of primer extersion fragments in a fragment mixture also containing template nucleic acids, said apparatus comprising:

> a polymer matrix (24) effective to preferentially retard the movement of the template nucleic acids through the matrix, when such a mixture is embedded in the matrix and an electric field is placed across the matrix;
> an, elongate electrophoretic medium (12, 14) effective to resolve such primer extension fragments, when an electric field is placed across the medium said medium having one end (20) in communication with said matrix (24), and means (32, 34, 36) for applying an electric field across the matrix and the medium, in a direction which draws the primer extension fragments through the matrix and into and through the medium.

21. Apparatus according to claim 20, further comprising first and a second containers (22, 28) wherein said matrix is held in said first container (22) and said medium is contained in a capillary tube (12) having one end submerged in said matrix in said first container and another end submerged in a buffar solution (30) in said second container (28).

22. Apparutus according to claim 20 or claim 21, wherein said polymer matrix is formed of a linear polymer having a molecular weight between $2 \times 10^5$ and $5 \times 10^6$ Daltons.

23. Apparatus according to any one of claims 20 to 22, wherein the matrix is dissolved in a denaturant-containing solution.

24. Apparatus according to claim 23, wherein the denaturant is selected from urea, formamide, 2-pyrrolidinone and n-methyl-2-pyrrolidinone.

**Patentansprüche**

1. Verfahren zum elektrophoretischen Trennen von Biomolekülen, das umfasst:

> a) eine Mischung von Biomolekülanalyten und Makrobiomolekülen mit einem größeren Molekulargewicht als dem der biomolekularen Analyten in einem Lösungsmittel vorzusehen;

> b) die Mischung in eine Polymermatrix einzubetten, die wirksam ist, um vorzugsweise die Bewegung der Makrobiomoleküle durch die Matrix zu hemmen, wenn ein elektrisches Feld über der Matrix vorgesehen wird;

> c) die Matrix mit der darin eingebetteten Mischung in Kommunikation mit einem Endbereich eines länglichen Elektrophoresemediums zu bringen, das wirksam zum Auflösen solcher Biomolekülanalyten ist, wenn ein elektrisches Feld über dem Medium vorgesehen wird;

> d) ein elektrisches Feld über der Matrix und dem Medium in einer Richtung anzulegen, die die Biomolekülanalyten durch die Matrix und in und durch das Medium zieht, wodurch eine Zunahme in dem Anteil von Biomolekülanalyten im Verhältnis zu in das Elektrophoresemedium eintretenden Makrobiomolekülen erreicht wird.

2. Verfahren nach Anspruch 1, bei dem die Makrobiomoleküle DNA-Sequenzierungsmatrizen sind.

3. Verfahren nach Anspruch 1 oder Anspruch 2, bei dem die Biomolekülanalyten Primerverlängerungsfragmente sind.

4. Verfahren nach einem der Ansprüche 1 bis 3, bei dem die Makrobiomoleküle ein Molekulargewicht von wenigstens 5000 Daltons haben.

5. Verfahren nach einem der Ansprüche 1 bis 4, bei dem das Polymer Hydroxyethylzellulose ist.

6. Verfahren zum Einfangen von DNA-Sequenzierungsmatrizen-Makromolekülen in einer Probenmischung, das die Schritte umfasst:

> a) eine Sequenzierungsprobenmischung vorzusehen, die wenigstens ein DNA-Matrizenmakromolekül mit einem Molekulargewicht größer als etwa $6 \times 10^5$ Daltons, ein DNA-Verlängerungsprodukt und ein Lösungsmittel enthält, und

> b) ein lineares Polymer in die Mischung einzubringen, das ein offenes verwickeltes Polymernetz in der Mischung bildet, welches die DNA-Matrize und die DNA-Verlängerungsprodukte darin einbettet, wobei das Netz Siebeigenschaften aufweist, die wirksam zum Hemmen von Bewegung der DNA-Matrize sind.

7. Verfahren zum Einbringen einer Probe in ein Kapillarröhrchen für Kapillarelektrophorese, das die Schritte umfasst:

> a) eine Probenmischung vorzusehen, die Makromoleküle und Analyten von Interesse in einem Lösungsmittel enthält;

> b) in die Mischung ein lineares Polymer mit einem Molekulargewicht einzuführen, das wirksam zum Bilden einer verwickelten, die Probenmischung darin einbettenden Polymermatrix ist, wobei die Matrix eine Maschengröße aufweist, die wirksam zum Hemmen von Bewegung der Makromoleküle durch die Matrix ist, wenn ein elektrisches Feld über der Matrix angelegt wird;

c) ein Ende eines mit Trennmedium gefüllten Kapillarelektrophoseröhrchens in die Probenmischung und Matrix einzuführen;

d) ein elektrisches Potential über der Probenmischung und dem Medium anzulegen, das wirksam zum Vergrößern des Anteils von Analyten von Interesse im Verhältnis zu in das Elektrophoresemedium eintretenden Makrobiomolekülen ist.

8. Verfahren nach Anspruch 7, bei dem die Makromoleküle Nucleinsäurematrizen sind.

9. Verfahren nach Anspruch 7 oder Anspruch 8, bei dem die Analyten von Interesse DNA-Verlängerungsprodukte enthalten.

10. Verfahren nach einem der Ansprüche 7 bis 9, bei dem die Matrix aus einem linearen Polymer mit einem Molekulargewicht zwischen $2 \times 10^5$ und $5 \times 10^6$ Daltons gebildet wird.

11. Kapillarelektrophoreseprobe für elektrophoretische Injektion in ein Kapillarelektrophorese-Trennmedium, wobei die Probe Makromoleküle und Analyten von Interesse in Lösung eingebettet in einer verwickelten Polymermatrix mit einer Maschengröße aufweist, die wirksam zum Hemmen von Bewegung der Makromoleküle in der Probe durch die Matrix ist, wenn ein elektrisches Feld über der -Matrix angelegt wird, um die Analyten durch die Matrix zu ziehen.

12. Probe nach Anspruch 11, bei der die Makromoleküle DNA-Sequenzierungsmatrizen darstellen.

13. Probe nach Anspruch 11 oder Anspruch 12, bei der die Analyten von Interesse DNA-Verlängerungsprodukte sind.

14. Probe nach einem der Ansprüche 11 bis 13, bei der die verwickelte Polymermatrix aus einem linearen Polymer mit einem Molekulargewicht zwischen $2 \times 10^5$ und $5 \times 10^6$ Daltons gebildet wird.

15. Verfahren zum Sequenzieren eines Nucleinsäurefragments, das umfasst:

a) eine Mischung von Primerverlängerungsfragmenten in einer Fragmentmischung vorzusehen, die auch Matrizennucleinsäuremoleküle enthält;

b) die Fragmentmischung in einer Polymermatrix einzubetten, die wirksam ist, um vorzugsweise die Bewegung der Matrizennucleinsäuremoleküle durch die Matrix zu hemmen, wenn ein elektrisches Feld über der Matrix vorgesehen wird;

c) die Matrix und die eingebettete Mischung in Kommunikation mit einem Endbereich eines länglichen elektrophoretischen Mediums vorzusehen, das wirksam zum Auflösen solcher Primerverlängerungsfragmente ist, wenn ein elektrisches Feld über dem Medium vorgesehen wird;

d) ein elektrisches Feld über der Matrix und dem Medium in einer Richtung anzulegen, die Primerverlängerungsfragmente durch die Matrix und in und durch das Medium zieht, wodurch eine Vergrößerung des Anteils von Primerverlängerungsfragmenten im Verhältnis zu in das Elektrophoresemedium eintretender Matrize erreicht wird.

16. Verfahren nach Anspruch 15, bei dem die verwikkelte Polymermatrix aus einem linearen Polymer mit einem Molekulargewicht von zwischen etwa $2 \times 10^5$ und $5 \times 10^6$ Daltons gebildet wird.

17. Verfahren nach einem der Ansprüche 3, 6, 9, 15 und 16, bei dem die Mischung/Probenmischung/Fragmentmischung weiter ein Denaturierungsmittel enthält.

18. Verfahren nach Anspruch 17, bei dem das Denaturierungsmittel ausgewählt ist von Urea, Formamid, 2-Pyrrolidinon und n-Methyl-2-pyrrolidinon.

19. Verfahren nach Anspruch 17 oder Anspruch 18, bei dem das Denaturierungsmittel eine wässerige Lösung mit einer Konzentration von 10 bis 70 Gewichtsprozent darstellt.

20. Elektrophoretisches Gerät (10) zur Verwendung beim Sequenzieren eines Nucleinsäurefragments durch elektrophoretische Trennung einer Mischung aus Primerverlängerungsfragmenten in einer Fragmentmischung, die auch Matrizennucleinsäuren enthält, wobei das Gerät umfasst:

eine Polymermatrix (24), die wirksam ist, um vorzugsweise die Bewegung der Matrizennucleinsäuren durch die Matrix zu hemmen, wenn eine solche Mischung in der Matrix eingebettet ist und ein elektrisches Feld über der Matrix vorgesehen wird;

ein längliches elektrophoretisches Medium (12, 14), das wirksam zum Auflösen solcher Primerverlängerungsfragmente ist, wenn ein elektrisches Feld über dem Medium vorgesehen wird, wobei das genannte Medium ein Ende (20) in Kommunikation mit der Matrix (24) aufweist; und

Mittel (32, 34, 36) zum Anlegen eines elektrischen Felds über der Matrix und dem Medium in einer Richtung, die die Primerverlängerungsfragmente durch die Matrix und in und durch das Medium zieht.

21. Gerät nach Anspruch 20, das weiter einen ersten und einen zweiten Behälter (22, 28) aufweist, und bei dem die Matrix in dem ersten Behälter (22) gehalten wird und das Medium in einem Kapillarröhrchen (12) enthalten ist, wobei ein Ende des Röhrchens in der Matrix in dem ersten Behälter und das andere Ende in einer Pufferlösung (30) in dem zweiten Behälter (28) untergetaucht ist.

22. Gerät nach Anspruch 20 oder Anspruch 21, bei dem die Polymermatrix aus einem linearen Polymer mit einem Molekulargewicht zwischen $2 \times 10^5$ und $5 \times 10^6$ Daltons gebildet ist.

23. Gerät nach einem der Ansprüche 20 bis 22, bei dem die Matrix in einer ein Denaturierungsmittel enthaltenden Lösung gelöst wird.

24. Gerät nach Anspruch 23, bei dem das Denaturierungsmittel von Urea, Formamid, 2-Pyrrolidinon und n-Methyl-2-pyrrolidinon ausgewählt ist.

## Revendications

1. Procédé de séparation électrophorétique de biomolécules, qui comprend les opérations consistant à :

a) se munir d'un mélange d'analytes biomoléculaires et de macrobiomolécules ayant une masse moléculaire plus grande que celle desdits analytes biomoléculaires dans un solvant ;
b) incorporer le mélange dans une matrice polymère permettant de retarder préférentiellement le déplacement des macrobiomolécules à travers la matrice quand un champ électrique est appliqué à travers la matrice ;
c) amener la matrice dans laquelle est incorporé le mélange, en communication avec une zone terminale d'un milieu d'électrophorèse allongé, permettant de séparer lesdits analytes biomoléculaires quand un champ électrique est appliqué à travers le milieu ;
d) appliquer un champ électrique à travers la matrice et le milieu dans une direction qui amène les analytes biomoléculaires à travers la matrice, et à travers et dans le milieu, ce qui réalise une augmentation de la proportion des analytes biomoléculaires par rapport aux macrobiomolécules pénétrant dans le milieu d'électrophorèse.

2. Procédé selon la revendication 1, dans lequel lesdites macrobiomolécules sont des matrices de séquençage d'ADN.

3. Procédé selon la revendication 1 ou 2, dans lequel lesdits analytes biomoléculaires sont des fragments d'allongement d'amorce.

4. Procédé selon l'une quelconque des revendications 1 à 3, dans lequel lesdites macrobiomolécules ont une masse moléculaire d'au moins 5 000 daltons.

5. Procédé selon l'une quelconque des revendications 1 à 4, dans lequel ledit polymère est l'hydroxyéthylcellulose.

6. Procédé pour piéger des macromolécules de matrice de séquençage d'ADN dans un mélange d'échantillons, qui comprend les étapes suivantes consistant à :

a) se munir d'un mélange d'échantillons de séquençage contenant au moins une macromolécule de matrice d'ADN ayant une masse moléculaire supérieure à environ $6 \times 10^5$ daltons, un produit d'allongement d'ADN et un solvant, et
b) introduire un polymère linéaire dans le mélange, en formulant un réseau de polymère enchevêtré ouvert dans le mélange contenant la matrice d'ADN et les produits d'allongement d'ADN, le réseau ayant des propriétés de tamisage permettant de retarder le déplacement de la matrice d'ADN.

7. Procédé pour charger un échantillon dans un tube capillaire pour électrophorèse sur capillaire, comprenant les étapes consistant à :

a) se munir d'un mélange d'échantillons contenant des macromolécules et des analytes présentant un intérêt dans un solvant ;
b) introduire, dans le mélange, un polymère linéaire ayant une masse moléculaire permettant de former une matrice de polymère enchevêtré contenant ledit mélange d'échantillons, ladite matrice ayant une granulométrie permettant de retarder le déplacement des macromolécules à travers la matrice quand un champ électrique est appliqué à travers la matrice ;

c) insérer, dans le mélange d'échantillons et la matrice, une extrémité d'un tube pour électrophorèse sur capillaire, rempli d'un milieu de séparation ;

d) appliquer un potentiel électrique à travers le mélange d'échantillons et le milieu, pour augmenter la proportion d'analytes présentant un intérêt, par rapport aux macrobiomolécules pénétrant dans le milieu d'électrophorèse.

8. Procédé selon la revendication 7, dans lequel lesdites macromolécules sont des matrices d'acide nucléique.

9. Procédé selon la revendication 7 ou 8, dans lequel lesdits analytes présentant un intérêt comprennent des produits d'allongement d'ADN.

10. Procédé selon l'une quelconque des revendications 7 à 9, dans lequel ladite matrice est formée d'un polymère linéaire ayant une masse moléculaire comprise entre $2 \times 10^5$ et $5 \times 10^6$ daltons.

11. Echantillon d'électrophorèse sur capillaire, pour injection électrophorétique dans un milieu de séparation pour électrophorèse sur capillaire, ledit échantillon comprenant des macromolécules et des analytes présentant un intérêt, en solution, incorporés dans une matrice de polymère enchevêtré ayant une granulométrie permettant de retarder le déplacement desdites macromolécules à l'intérieur dudit échantillon à travers la matrice quand un champ électrique est appliqué à travers la matrice pour amener lesdits analytes à travers ladite matrice.

12. Echantillon selon la revendication 11, dans lequel lesdites macromolécules sont des matrices de séquençage d'ADN.

13. Echantillon selon la revendication 11 ou 12, dans lequel lesdits analytes présentant un intérêt sont des produits d'allongement d'ADN.

14. Echantillon selon l'une quelconque des revendications 11 à 13, dans lequel ladite matrice de polymère enchevêtré est formée d'un polymère linéaire ayant une masse moléculaire comprise entre $2 \times 10^5$ et $5 \times 10^6$ daltons.

15. Procédé pour séquencer un fragment d'acide nucléique, qui comprend les opérations consistant à:

a) se munir d'un mélange de fragments d'allongement d'amorce dans un mélange de fragments contenant aussi des molécules d'acide nucléique de matrice ;

b) incorporer le mélange de fragments dans une matrice de polymère pour retarder d'une manière préférentielle le déplacement des molécules d'acide nucléique de matrice à travers la matrice, quand un champ électrique est appliqué à travers la matrice ;

c) amener la matrice et du mélange incorporé en communication avec une zone terminale d'un milieu d'électrophorèse allongé, pour séparer lesdits fragments d'allongement d'amorce quand un champ électrique est appliqué à travers le milieu ;

d) appliquer un champ électrique à travers la matrice et le milieu, dans une direction qui amène les fragments d'allongement d'amorce à travers la matrice, et à travers et dans le milieu, ce qui réalise une augmentation de la proportion des fragments d'allongement d'amorce par rapport à la matrice pénétrant dans le milieu d'électrophorèse.

16. Procédé selon la revendication 15, dans lequel ladite matrice de polymère enchevêtré est formée d'un polymère linéaire ayant une masse moléculaire comprise entre environ $2 \times 10^5$ et $5 \times 10^6$ daltons.

17. Procédé selon l'une quelconque des revendications 3, 6, 9, 15 et 16, dans lequel le mélange/mélange d'échantillons/mélange de fragments contient en outre un dénaturant.

18. Procédé selon la revendication 17, dans lequel le dénaturant est choisi parmi l'urée, le formamide, la 2-pyrrolidinone et la N-méthyl-2-pyrrolidinone.

19. Procédé selon la revendication 17 ou 18, dans lequel le dénaturant est en solution aqueuse, à une concentration de 10 à 70 % en poids.

20. Appareil électrophorétique (10) pour utilisation dans le séquençage d'un fragment d'acide nucléique par séparation électrophorétique d'un mélange de fragments d'allongement d'amorce dans un mélange de fragments contenant aussi des acides nucléiques de matrice, ledit appareil comprenant :

une matrice de polymère (24) permettant de retarder préférentiellement le déplacement des acides nucléiques de matrice à travers la matrice, quand un tel mélange est incorporé dans la matrice et qu'un champ électrique est appliqué à travers la matrice ;

un milieu d'électrophorèse allongé (12, 14) permettant de séparer lesdits fragments d'allongement d'amorce quand un champ électrique est appliqué à travers le milieu, ledit milieu ayant une extrémité (20) communiquant avec ladite matrice (24) ; et

des moyens (32, 34, 36) pour appliquer un champ électrique à travers la matrice et le milieu, dans une direction qui amène les fragments d'allongement d'amorce à travers la matrice et dans et à travers le milieu.

21. Appareil selon la revendication 20, qui comprend en outre un premier et un deuxième réservoirs (22, 28), où ladite matrice est contenue dans ledit premier réservoir (22) et ledit milieu est contenu dans un tube capillaire (12) ayant une extrémité immergée dans ladite matrice se trouvant dans ledit premier réservoir et une autre extrémité immergée dans une solution tampon (30) se trouvant dans ledit deuxième réservoir (28).

22. Appareil selon la revendication 20 ou 21, dans lequel ladite matrice de polymère est formée d'un polymère linéaire ayant une masse moléculaire comprise entre d'environ $2 \times 10^5$ et $5 \times 10^6$ daltons.

23. Appareil selon l'une quelconque des revendications 20 à 22, dans lequel la matrice est dissoute dans une solution contenant un dénaturant.

24. Appareil selon la revendication 23, dans lequel le dénaturant est choisi parmi l'urée, le formamide, la 2-pyrrolidinone et la N-méthyl-2-pyrrolidinone.

Fig. 1

# Fig. 2

**Fig. 3**

Fig. 4

**Fig. 5**

EP 0 680 605 B1

Fig. 6

EP 0 680 605 B1